Europäisches Patentamt

European Patent Office

Office européen des brevets

(11) Numéro de publication: **0 247 946 B1**

# FASCICULE DE BREVET EUROPEEN

(45) Date de publication de fascicule du brevet:
**11.12.91**

(51) Int. Cl.⁵: **A61L 9/01, A61L 11/00**

(21) Numéro de dépôt: **87401204.0**

(22) Date de dépôt: **27.05.87**

Le dossier contient des informations techniques présentées postérieurement au dépôt de la demande et ne figurant pas dans le présent fascicule.

(54) **Procédé de neutralisation des odeurs désagréables des excrétions et excréments des animaux.**

(30) Priorité: **29.05.86 FR 8607856**

(43) Date de publication de la demande:
**02.12.87 Bulletin 87/49**

(45) Mention de la délivrance du brevet:
**11.12.91 Bulletin 91/50**

(84) Etats contractants désignés:
**AT BE CH DE ES FR GB GR IT LI LU NL SE**

(56) Documents cités:
**EP-A- 0 005 618**
**DE-C- 458 192**
**FR-A- 1 590 898**
**FR-A- 2 416 016**

**KIRK OTHMER; Encyclopedia of Chemical Technology, Third Edition, vol. 16, pp. 297-305**

(73) Titulaire: **ROBERTET S.A.**
**37, avenue Sidi-Brahim**
**F-06130 Grasse(FR)**

(72) Inventeur: **Joulain, Daniel**
**Cybèle Résidences Chemin de la Cavalerie**
**F-06130 Grasse(FR)**
Inventeur: **Racine, Philippe**
**La Pastorale Quartier Condamine**
**F-06740 Chateauneuf de Grasse(FR)**
Inventeur: **Maire, Françoise**
**19, Boulevard Victor-Hugo**
**F-92200 Neuilly sur Seine(FR)**

(74) Mandataire: **Rinuy, Santarelli**
**14, avenue de la Grande Armée**
**F-75017 Paris(FR)**

## Description

La présente invention concerne un procédé efficace pour lutter contre et neutraliser les odeurs désagréables d'excrétions et excréments des animaux.

Il est connu qu'il est très difficile de se débarrasser des odeurs désagréables dues à des excrétions animales notamment dans le cas des animaux domestiques comme en particulier les urines et excréments de chats ou bien des odeurs des excrétions d'animaux d'élevage tels que les porcins, les bovins, les ovins, les lapins, volailles et autres animaux de basse-cour.

Dans le cas particulier des urines et excréments de chats, leur odeur est particulièrement tenace et persistante. Il s'avère que quel que soit l'agent du type parfum ou déodorant que l'on utilise pour combattre ces odeurs désagréables, celles-ci réapparaissent au bout d'un certain temps, une fois que l'odeur propre au parfum ou au déodorant a elle-même disparu par évaporation. Par ailleurs, dans les centres d'élevage d'animaux tels que porcins, bovins, ovins, lapins, les odeurs émanant des étables ou des locaux occupés par ces animaux ne sont en général pas combattues, faute de moyens efficaces.

La demande de brevet européen EP-A-0.005.618 décrit un produit déodorant comprenant en tant que constituant actif une composition dont les composants sont choisis parmi six familles de substances en proportions déterminées, utilisables en particulier en tant que savons, déodorants et déodorants corporels.

La demande de brevet français FR-A-1.590.898, pour sa part, décrit la possibilité de recouvrir les mauvaises odeurs de toute origine pendant une durée prolongée grâce à un support imprégné d'un agent parfumant, lequel support est également imprégné d'un agent retardateur d'évaporation.

Or la présente invention fournit justement un procédé ayant pour effet soit d'éviter le développement d'une odeur après que les animaux ont déversé ou déposé leurs excrétions ou leurs excréments sur leurs litières, leurs lisiers ou toute autre surface et ce, à titre préventif, soit de lutter efficacement en neutralisant des odeurs désagréables qui se sont déjà manifestées et installées soit sur une surface, soit dans un local par suite du dépôt des excrétions ou excréments des animaux aussi bien domestiques que d'élevage ou de l'accumulation de ces excrétions ou excréments.

La présente invention a donc pour objet un procédé de neutralisation des odeurs désagréables des excrétions et excréments des animaux caractérisé en ce que l'on applique sur les surfaces, à la source des odeurs désagréables, une dose efficace d'un agent actif appartenant à la famille des composés utilisés en parfumerie pour leurs propriétés odoriférantes mais présentant en outre une tension de vapeur inférieure ou égale à 4 Pa à 25°C, l'agent actif étant utilisé tel quel ou en solution dans un solvant approprié ne renfermant pas de matériau abrasif, agent blanchissant, cire ou filmogène.

L'invention couvre également une formulation particulière comme cela ressortira plus clairement de la description qui va suivre.

De façon générale, on citera comme composés pouvant convenir selon l'invention, les composés choisis parmi les composés suivants :

- les alcools aliphatiques, avantageusement de $C_{10}$ à $C_{12}$, comme le décanol, le citronellol, le géraniol ;
- les aldéhydes, avantageusement de $C_{10}$ à $C_{13}$, soit aliphatiques comme le dodécanal normal ou ramifié, l'aldéhyde myrac, soit aromatiques comme l'aldéhyde cyclamen, l'hélional, l'héliotropine, le paraméthylphénylacétaldéhyde, la vanilline et ses dérivés ;
- les cétones aliphatiques, avantageusement en $C_{13}$ et $C_{14}$, comme les $\alpha$ et $\beta$-ionones et damascones, ainsi que les cétones aliphatiques et aromatiques à odeur musquée pouvant avoir jusqu'à 18 atomes de carbone ;
- les esters aliphatiques, avantageusement en $C_8$ à $C_{15}$, comme le dihydro-jasmonate de méthyle, le jasmonate de méthyle, le cinnamate de méthyle, le méthylphénylglycidate d'éthyle, les esters aromatiques comme l'anthranilate de méthyle, le N-méthylanthranilate de méthyle, le phénylacétate de p-crésyle, le salicylate d'amyle ;
- les lactones aromatiques comme la coumarine, la dihydrocoumarine et les lactones aliphatiques comme la gamma-décalactone, dodécalactone et undécalactone ;
- les phénols comme l'eugénol et l'isoeugénol ;
- les éthers aromatiques comme l'oxyde de diphényle, les éthers méthylés et éthylés du naphtol, le galaxolide ;
- les composés azotés du type amines comme l'indole et ses composés de réaction avec l'hydroxycitronellal dénommés indolène et les nitriles aliphatiques comme le tridécène-2-nitrile ;
- les amines aromatiques y compris les dérivés pyridiniques comme la (méthyl-2'-pentène-2'-yl)-2-méthyl-5-pyridine.

Parmi les composés mentionnés ci-dessus convenant selon l'invention, on citera de préférence la coumarine, l'allyl-ionone, l'eugénol et l'isoeugénol, le dihydrojasmonate de méthyle, l'indole et l'indolène, l'hélional, l'alpha et la béta ionone et la (méthyl-2'-pentène-2'-yl)-2-méthyl-5-pyridine.

Ceux des composés qui sont liquides peuvent être utilisés tels quels. Par contre, les composés cristallisés seront utilisés après leur dissolution dans leur solvant habituel bien connu en parfumerie comme le phtalate d'éthyle, le benzoate de benzyle, le citrate d'éthyle et analogues.

En outre, si on le désire, les composés mis en oeuvre dans le procédé selon l'invention peuvent aussi être utilisés en mélanges dans des proportions variables donnant une formule n'ayant pratiquement aucun effet olfactif désagréable.

Rien n'empêche toutefois d'ajouter aux composés mis en oeuvre dans le procédé selon l'invention, pris seuls ou en mélanges, et à volonté d'autres matières premières parfumantes agrémentant pour l'usager et pour l'environnement l'effet olfactif de la formulation à appliquer.

Les exemples suivants sont donnés à titre illustratif et nullement limitatif du genre de formulations que l'on peut élaborer et donnant des résultats sur la neutralisation préventive ou effective des odeurs désagréables dues aux excréments et/ou dépôts et/ou à l'accumulation des excrétions animales. Bien entendu, et conformément à l'invention, les composés actifs comme l'eugénol la coumarine, l'allylionone, le dihydrojasmonate de méthyle, l'indolène ou l'hélional qui figurent dans les exemples de formulations données ci-dessous peuvent être utilisés isolément. Les autres ingrédients utilisés l'ont été soit à titre d'agents odoriférants, soit à titre de solvant comme le phtalate d'éthyle désigné par P.E.

Exemple 1
Formulation I (F₁)

On mélange, pour 1000 g de formulation, les composés suivants :
- citral 38 g
- acétate de linalyle 114 g
- diéthylacétal du citral 38 g
- eugénol 51 g
- linalol 228 g
- acétate d'hexényle à 10 % dans P.E. 51 g
- P.E. q.s. à 1000 g

Exemple 2
Formulation II (F₂)

Comme dans l'exemple 1, on mélange les composés suivants (pour 1000 g de formulation) :
- coumarine 25 g
- allylionone 38 g
- dihydrojasmonate de méthyle 25 g
- indolène 25 g
- (hydroxy-4-phényl)-4-butanone-2 à 1 % dans P.E. 13 g
- hélional 10 g

- aldéhyde-alpha-hexylcinnamique 253 g
- P.E. q.s. à 1000 g

Appliqués en solution dans un solvant approprié, par exemple à la dose de 6 %, les composés utilisés dans l'invention se sont avérés être particulièrement actifs aussi bien pour prévenir le développement ou le dégagement d'odeurs désagréables lorsqu'on s'attend à ce que les animaux soient amenés à répandre leurs excrétions ou déposer leurs excréments en des endroits pouvant leur être destinés à cet effet, que pour faire disparaitre, annihiler ou détruire les odeurs qui se seraient déjà développées en ces mêmes endroits ou même en d'autres endroits lorsque, accidentellement, ces mêmes animaux se sont libérés de ces excrétions ou ont déposé leurs excréments.

C'est ainsi en particulier, qu'à titre préventif, on a pulvérisé l'une ou l'autre des formulations F₁ et F₂ ci-dessus sur des litières de chats. Que cette pulvérisation soit répétée ou non, on constate que l'effet de chaque pulvérisation est durable, que les litières ainsi traitées n'ont pas d'effet répulsif sur l'animal et que, par voie de conséquence, on réalise une économie appréciable de la quantité du produit constitutif desdites litières comme par exemple la sciure, la perlite et analogues. La neutralisation des odeurs est pratiquement totale d'où disparition de tous les désagréments qui en résultent. De même, la ou les mêmes pulvérisations effectuées sur des litières déjà souillées font disparaître les odeurs désagréables résultant du dépôt des excrétions ou excréments entre deux changements de litières.

Exemple 3

On a procédé aux expériences suivants : on a utilisé de l'attapulgite du commerce pour litière de chat et on l'a lavée à plusieurs reprises avec du chlorure de méthylène purifié. Après élimination de tout solvant résiduel, cette attapulgite a été considérée comme prête à l'emploi.

En utilisant un groupe donné d'animaux (mâles castrés ou non, femelles), on a recueilli l'attapulgite imprégnée fortement d'urine de ces chats et on l'a soumise à l'extraction par du chlorure de méthylène purifié.

Après élimination du solvant d'extraction par distillation sous pression ordinaire et en utilisant une colonne à garnissage de raschig de 150 cm de longueur, on a obtenu un mélange brut de couleur brune et d'odeur typique et extrêmement désagréable.

Examiné en chromatographie en phase gazeuse au moyen d'une colonne capillaire en silice fondue de 50 m de longueur et de diamètre 0,32 mm, recouverte de silicone SE 30, ce mélange s'avère composé de nombreux constituants (figure I). L'examen analytique est approfondi par l'utilisation du couplage chromatographie en phase gazeuse-spectrométrie de masse et du couplage chromatographie en phase gazeuse-spectrocopie infrarouge à transformée de Fourier.

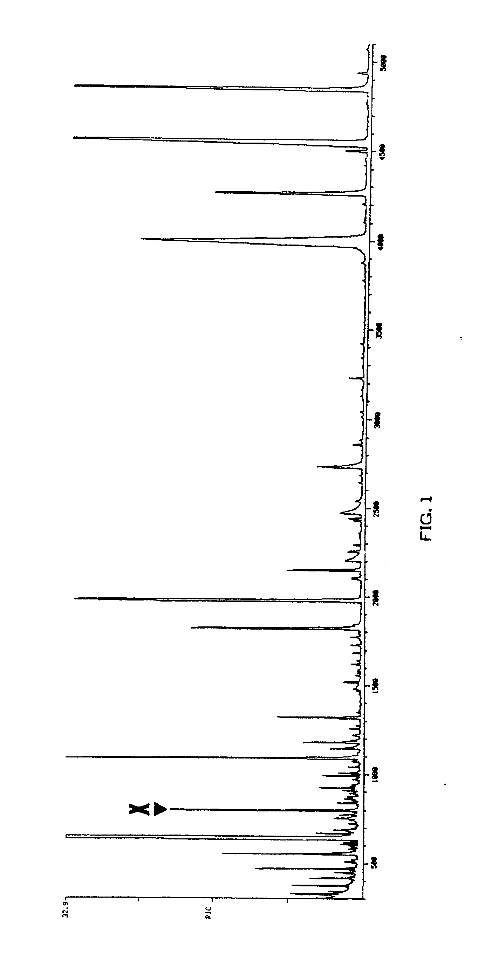

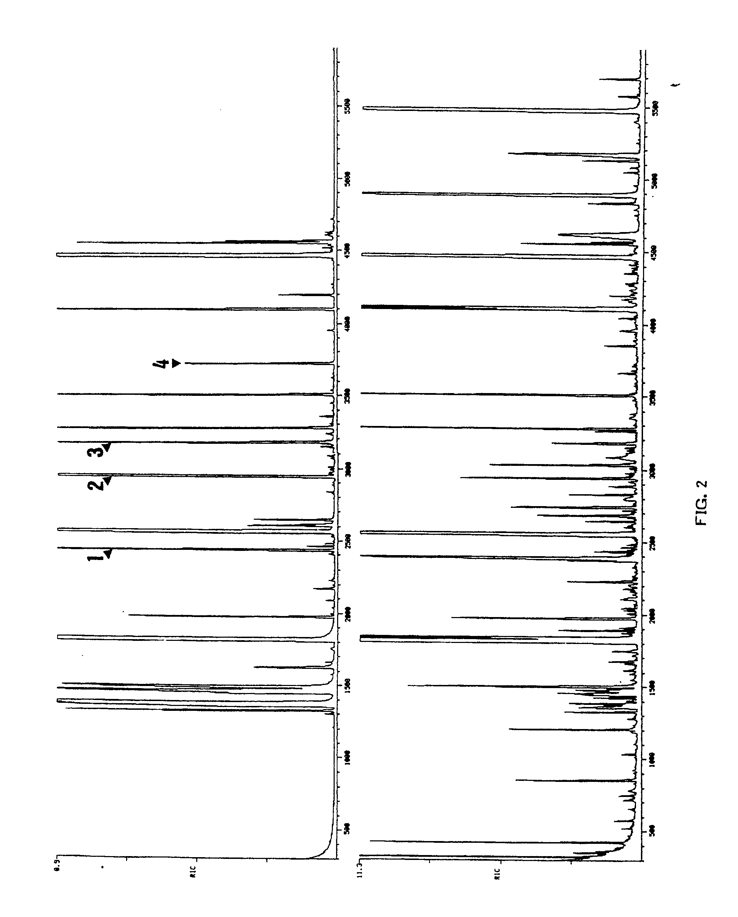

Par examen olfactif de chaque constituant correspondant à chaque pic chromatographique, en utilisant cette fois une colonne de 30 m de longueur et de 0,53 mm de diamètre et de même polarité, on note la présence de plusieurs constituants très malodorants présentant des odeurs de type sulfuré, fétide, de fumée, de sueur, de légumes avariés, etc. En particulier, il est un constituant X, noté par une flèche sur la figure I, qui présente à un degré extrême ment prononcé la plupart de ces qualificatifs.

Comme dans les exemples précédents, on a pulvérisé une formulation $F_3$ contenant entre autres et notamment les produits suivants : citral, eugénol, coumarine et hélional.

La litière ainsi traitée et "visitée" longuement par les mêmes animaux que précédemment, ainsi que de la litière témoin imprégnée de la même façon de la formulation $F_3$, mais non souillée par les animaux, ont été soumises dans les mêmes conditions que précédemment à l'extraction par le chlorure de méthylène purifié.

Dans chaque cas, il a été obtenu un extrait brut qui a été analysé par chromatographie en phase gazeuse et par spectrométrie de masse couplée.

Dans la figure II, le chromatogramme supérieur correspond à l'extrait brut de la litière imprégnée de la formulation $F_3$ mais non souillée d'urine ; les flèches 1, 2, 3 et 4 indiquent la présence du Z-citral, de l'eugénol, de la coumarine et de l'hélional respectivement. Le chromatogramme inférieur correspond à l'extrait brut de litière imprégnée de la formulation $F_3$ et souillée d'urine.

Dans ces conditions, on observe les phénomènes significatifs suivants :

- disparition totale dans la litière souillée d'urine, du pic X attribué à la substance très malodorante;
- disparition concomittante de l'hélional et diminution sensible des proportions du Z-citral, de la coumarine et de l'eugénol notamment (ces variations ne sont d'ailleurs pas limitées à ces seuls produits) ;
- en outre, l'extrait issu de la litière souilléé d'urine ne présente pas d'odeur particulièrement désagréable.

Par ailleurs, on notera qu'en cas d'accident, c'est-à-dire au cas où l'animal aurait souillé d'autres surfaces, lisses ou non, telles que surfaces métalliques, tissus, tapis, coussins et analogues, la ou les pulvérisations desdites formulations supprime(nt) entièrement l'odeur désagréable qui pourrait persister après un nettoyage desdites surfaces.

Une des autres applications intéressantes des agents mis en oeuvre dans le procédé selon l'invention réside dans les milieux vétérinaires. On a en effet constaté que malgré toutes les précautions et les moyens que l'on utilise actuellement pour soit désinfecter, soit essayer de désodoriser les plans de travail des cabinets de vétérinaires, les animaux qui se succèdent dans lesdits cabinets sont sensibles aux odeurs résiduelles du passage de l'animal précédant leur arrivée dans le cabinet de consultation proprement dit.

C'est ainsi que les vétérinaires savent très bien qu'un chien pénétrant dans leur cabinet sécrète, sous l'effet probablement de la peur, par leurs glandes anales, une substance à très forte odeur et très désagréable qui est actuellement très difficile à neutraliser.

Or les produits mis en oeuvre dans le procédé selon l'invention, lorsqu'ils sont pulvérisés à titre préventif sur les parties anales et péri-anales des chiens, neutralisent totalement l'effet résultant de cette sécrétion des glandes en cause. De même, les plans de travail et d'examen des cabinets vétérinaires, qu'ils soient métalliques ou non, lorsqu'ils sont pulvérisés au moyen des produits mis en oeuvre dans le procédé selon l'invention, annihilent totalement les effets désagréables dûs à ces odeurs animales, que cette odeur soit due à leurs excrétions ou au simple contact physique de l'animal sur les surfaces d'examen.

On a également constaté que les produits mis en oeuvre dans le procédé selon l'invention sont aussi efficaces lorsqu'ils sont pulvérisés sur d'autres litières d'animaux comme les lisiers de porcins, les litières de lapins ou d'ovins, étables, basses-cours, cages à oiseaux, volières, etc.

Comme précédemment signalé, ces produits peuvent être utilisés seuls ou en mélanges entre eux et/ou avec d'autres agents odoriférants suivant les résultats que l'on désire obtenir pour améliorer une atmosphère ambiante. Ces produits, seuls ou en mélanges, peuvent être simplement utilisés dans leur solvant si ces produits sont à l'état solide ou avec des solvants facilitant l'application ou l'homogénéité. Les proportions sont elles-mêmes variables suivant les cas et l'intensité de l'odeur à combattre.

## Revendications

1. Procédé de neutralisation des odeurs désagréables des excrétions et excréments des animaux caractérisé en ce que l'on traite les surfaces, à la source des odeurs désagréables, à l'aide d'une dose efficace d'un agent actif appartenant à la famille des composés utilisés en parfumerie pour leurs propriétés odoriférantes mais présentant en outre une tension de vapeur inférieure ou égale à 4 Pa à 25°C, l'agent actif étant utilisé tel quel ou en solution dans un solvant approprié ne renfermant pas de matériau abrasif, agent blanchissant, cire ou filmogène.

2. Procédé selon la revendication 1, caractérisé en ce que l'agent actif est choisi parmi les composés suivants : les alcools aliphatiques, avantageusement de $C_{10}$ à $C_{12}$, comme le décanol ; les aldéhydes, avantageusement de $C_{10}$ à $C_{13}$, soit aliphatiques comme le dodécanal normal ou ramifié, l'aldéhyde myrac, soit aromatiques comme l'aldéhyde cyclamen, l'hélional, l'héliotropine, le paraméthylphénylacétaldéhyde, la vanilline et ses dérivés ; les cétones aliphatiques, avantageusement en $C_{13}$ et $C_{14}$, comme les $\alpha$ et $\beta$-ionones et damascones, ainsi que les cétones aliphatiques et aromatiques à odeur musquée pouvant avoir jusqu'à 18 atomes de carbone ; les esters aliphatiques, avantageusement en $C_8$ à $C_{15}$, comme le dihydro-jasmonate de méthyle, le jasmonate de méthyle, le cinnamate de méthyle, le méthylphénylglycidate d'éthyle, les esters aromatiques comme l'anthranilate de méthyle, le N-méthylanthranilate de méthyle, le phénylacétate de p-crésyle, le salicylate d'amyle ; les lactones aromatiques comme la coumarine, la dihydrocoumarine et les lactones aliphatiques comme la gamma-décalactone, dodécalactone et undécalactone ; les phénols comme l'eugénol et l'isoeugénol ; les éthers aromatiques comme l'oxyde de diphényle, les éthers méthylés et éthylés du naphtol, le galaxolide ; les composés azotés du type amines comme l'indole et ses composés de réaction avec l'hydroxycitronellal dénommés indolène et les nitriles aliphatiques comme le tridécène-2-nitrile ; les amines aromatiques y compris les dérivés pyridiniques comme la (méthyl-2'-pentène-2'-yl)-2-méthyl-5pyridine.

3. Procédé selon l'une des revendications 1 ou 2, caractérisé en ce que l'agent actif est choisi parmi les coumarine, allyl-ionone, eugénol et isoeugénol, dihydrojasmonate de méthyle, indole et indolène, hélional, $\alpha$ et $\beta$-ionone et (méthyl-2'-pentène-2'-yl)-2-méthyl-5-pyridine.

4. Procédé selon l'une quelconque des revendications 1 à 3 pour la prévention ou la lutte contre le développement ou la manifestation d'odeurs désagréables provenant des excrétions ou excréments domestiques, de bovins, d'ovins, de porcins, de lapins, de volailles et autres animaux de basse-cour et de volières.

5. Procédé selon la revendication 4, caractérisé en ce que, lorsque l'agent actif est liquide, il est utilisé tel quel, alors que s'il est cristallisé, il est utilisé après dissolution dans un solvant approprié connu en parfumerie, choisi parmi le phtalate d'éthyle, le benzoate de benzyle, le citrate d'éthyle et analogues.

6. Procédé selon l'une des revendications 4 ou 5, caractérisé en ce qu'on utilise l'agent actif seul ou en mélange avec des agents actifs autres que ceux définis à la revendication 1 et en outre avec une ou plusieurs matières premières parfumantes.

7. Procédé selon l'une quelconque des revendications 1 à 6 pour prévenir ou lutter contre les manifestations désagréables dues aux odeurs inhérentes aux excrétions et excréments d'animaux domestiques ou d'élevage par pulvérisation ou étalement sur toute surface recevant ou ayant reçu lesdits excrétions ou excréments.

8. Procédé selon l'une quelconque des revendications 1 à 7, caractérisé en ce qu'il est réalisé par pulvérisation.

9. Composition caractérisée en ce qu'elle renferme un mélange de citral, d'eugénol, de coumarine et d'hélional.

**Claims**

1. Neutralization process for disagreeable odours from animal excretions and excrement characterized in that the surfaces, at the source of the disagreeable odours, are treated using an effective dose of an active agent belonging to the group of compounds used in the perfume industry for their fragrant properties but having, in addition, a vapour pressure of less than or equal to 4 Pa at 25°C, the active agent being used as it is or in solution in an appropriate solvent which does not contain abrasive material, bleaching agents, wax or film-forming agents.

2. Process according to claim 1, characterized in that the active agent is chosen from the following compounds: aliphatic alcohols, advantageously from $C_{10}$ to $_{12}$, such as decanol; aldehydes, advantageously from $C_{10}$ to $C_{13}$, either aliphatics such as normal or branched dodecanal, myrac aldehyde or aromatics such as cyclamen aldehyde, helional, heliotropine, para-methylphenylacetaldehyde, vanillin and its derivatives; aliphatic ketones, advantageously $C_{13}$ and $C_{14}$, such as alpha- and beta- ionones and damascones, as well as aliphatica and aromatic ketones having a musk-like odour having up to 18 carbon atoms; aliphatic esters advantageously from $C_8$ to $C_{15}$, such as methyl dihydrojasmonate, methyl jasmonate, methyl cinnamate, ethyl methylphenylglycidate, aromatic esters such as methyl anthranilate, methyl N-methylanthranilate, p-cresyl phenylacetate, amyl salicylate; aromatic lactones such as coumarin, dihydrocoumarin and aliphatic lactones such as gamma-decalactone, dodecalactone and undecalactone; phenols such as eugenol and isoeugenol; aromatic ethers such as diphenyl oxide, methyl and ethyl ethers of naphthol, galaxolide; nitro compounds of the amine type, such as indole and its reaction compounds with hydroxycitronellal called indolene and aliphatic nitriles, such as tridecene-2-nitrile; aromatic amines including pyridinic derivatives such as 2-(2'-methyl-pent-2'-enyl)-5-methyl- pyridine.

3. Process according to one of claims 1 or 2, characterized in that the active agent is chosen from coumarin, allylionone, eugenol and isoeugenol, methyl dihydrojasmonate, indole and indolene, helional, alpha- and beta-ionone and 2-(2'-methyl-pent-2'-enyl)-5-methyl-pyridine.

4. Process according to any one of claims 1 to 3 for preventing or combatting the development or manifestation of disagreeable odours resulting from excretions or excrement of domestic animals, cattle, sheep, pigs, rabbits, poultry and other farmyard and aviary animals.

5. Process according to claim 4, characterized in that, when the active agent is liquid, it is used as it is, whereas if it is crystalline, it is used after dissolution in an appropriate solvent well-known in the perfume industry, chosen from ethyl phthalate, benzyl benzoate, ethyl citrate and their analogues.

6. Process according to one of claims 4 or 5, characterized in that the active agent is used alone or as a mixture with active agents other than those defined in claim 1 and in addition with one or more primary perfuming substances.

7. Process according to any one of claims 1 to 6 for preventing or combating manifestations of disagreeable odours due to the inherent odours of excretions and excrement of domestic or farm animals by spraying or spreading over all the surface receiving or having received said excretions or excrement.

8. Process according to any one of claims 1 to 7, characterized in that it is carried out by spraying.

9. Composition characterized in that it contains a mixture of citral, eugenol, coumarin and helional.

## Patentansprüche

1. Verfahren zur Neutralisierung unangenehmer Gerüche von tierischen Ausscheidungen und Exkrementen, dadurch gekennzeichnet, daß die Oberflächen an der Quelle der unangenehmen Gerüche mit Hilfe einer wirksamen Dosis eines aktiven Mittels, das zur Gruppe der bei der Parfümherstellung auf Grund ihrer Dufteigenschaften verwendeten Verbindungen gehört, jedoch außerdem einen Dampfdruck von 4 Pa oder weniger bei 25°C aufweist, behandelt werden, wobei das an sich oder in Lösung in einem geeigneten Lösungsmittel verwendete aktive Mittel keinerlei Schleifmaterialien, Bleichmittel, Wachs oder Filmbildner enthält.

2. Verfahren nach Anspruch 1, dadurch gekennzeichnet, daß das aktive Mittel aus den folgenden Verbindungen: aliphatischen Alkoholen, vorteilhaft mit 10 bis 12 Kohlenstoffatomen, wie Decanol; Aldehyden, vorteilhaft mit 10 bis 13 Kohlenstoffatomen, entweder aliphatisch, wie n-Dodecanal, oder verzweigtkettig, Myracaldehyd, entweder aromatisch, wie Cyclamenaldehyd, Helional, Heliotropin, p-Methylphenylacetaldehyd, Vanillin und dessen Derivaten; aliphatischen Ketonen, vorteilhaft mit 13 bis 14 Kohlenstoffatomen, wie α- und ß-Iononen und Damasconen, sowie aliphatischen und aromatischen Ketonen mit Moschusgeruch, die bis zu 18 Kohlenstoffatome aufweisen können; aliphatischen Estern, vorteilhaft mit 8 bis 15 Kohlenstoffatomen, wie Methyldihydrojasmonat, Methyljasmonat, Methylcinnamat, Methylphenylethylglycidat, aromatischen Estern, wie Methylanthranilat, Methyl-N-methylanthranilat, p-Cresylphenylacetat, Amylsalicylat; aromatischen Lactonen, wie Cumarin, Dihydrocumarin, und den aliphatischen Lactonen, wie Gammadecalacton, Dodecalacton und Undecalacton; Phenolen, wie Eugenol und Isoeugenol; aromatischen Ethern, wie Diphenyloxid, methylierten und ethylierten Naphtholethern, Galaxolid; Stickstoffverbindungen vom Amintyp, wie Indol und dessen Reaktionsverbindungen mit Hydroxycitronellal, die als Indolen bezeichnet werden, und aliphatischen Nitrilen, wie Tridecen-2-nitril; den aromatischen Aminen einschließlich Pyridinderivate, wie 2-(2'-Methylpent-2'-enyl)-5-methylpyridin, ausgewählt ist.

3. Verfahren nach einem der Ansprüche 1 oder 2, dadurch gekennzeichnet, daß das aktive Mittel aus Cumarin, Allylionon, Eugenol und Isoeugenol, Methyldihydrojasmonat, Indol und Indolen, Helional, α- und ß-Ionon und 2-(2'-Methylpent-2'-enyl)-5-methylpyridin ausgewählt ist.

4. Verfahren nach einem der Ansprüche 1 bis 3, zur Verhütung oder Bekämpfung der Entwicklung oder Manifestation unangenehmer Gerüche, die von Ausscheidungen oder Exkrementen von Haustieren, Rindern, Schafen, Schweinen, Kaninchen, Geflügel, und anderem Kleinvieh und in Volieren gehaltenen Vögeln stammen.

5. Verfahren nach einem der Ansprüche 1 bis 4, dadurch gekennzeichnet, daß das aktive Mittel, wenn es flüssig ist, an sich verwendet wird, und wenn es kristallisiert ist, nach Lösen in einem bei der Parfümherstellung bekannten geeigneten Lösungsmittel, ausgewählt aus Ethylphthalat, Benzylbenzoat, Ethylcitrat und Analoga, verwendet wird.

6. Verfahren nach einem der Ansprüche 4 oder 5, dadurch gekennzeichnet, daß ein aktives Mittel allein oder gemischt mit anderen als oben in Anspruch 1 definierten aktiven Mitteln und außerdem mit einem oder mehreren Parfümausgangsmaterialien verwendet wird.

7. Verfahren nach einem der Ansprüche 1 bis 6, zur Verhütung oder Bekämpfung unangenehmer Manifestationen auf Grund von in Ausscheidungen oder Exkrementen von Haus- oder Zuchttieren inhärenten Gerüchen durch Sprühen oder Verteilen auf die gesamte Oberfläche, die die Ausscheidungen oder Exkremente aufnimmt oder aufgenommen hat.

8. Verfahren nach einem der Ansprüche 1 bis 7, dadurch gekennzeichnet, daß es durch Sprühen durchgeführt wird.

9. Zusammensetzung, dadurch gekennzeichnet, daß sie eine Mischung von Citral, Eugenol, Cumarin und Helional enthält.

FIG. 1

FIG. 2